(19) **Europäisches Patentamt**
**European Patent Office**
**Office européen des brevets**

(11) **EP 1 958 665 B1**

(12) **EUROPEAN PATENT SPECIFICATION**

(45) Date of publication and mention
of the grant of the patent:
**25.08.2010 Bulletin 2010/34**

(51) Int Cl.:
*A61N 5/10* (2006.01)    *G01T 1/04* (2006.01)
*G01T 1/169* (2006.01)

(21) Application number: **08250511.6**

(22) Date of filing: **12.02.2008**

(54) **Ionizing radiations**

Ionisierende Strahlungen

Radiations ionisantes

(84) Designated Contracting States:
**AT BE BG CH CY CZ DE DK EE ES FI FR GB GR
HR HU IE IS IT LI LT LU LV MC MT NL NO PL PT
RO SE SI SK TR**

(43) Date of publication of application:
**20.08.2008 Bulletin 2008/34**

(73) Proprietors:
• **Aljohani, Mohammed
Jeddah 21589 (SA)**
• **Djouider, Fathi
Jeddah 21589 (SA)**

(72) Inventors:
• **Aljohani, Mohammed
Jeddah 21589 (SA)**

• **Djouider, Fathi
Jeddah 21589 (SA)**

(74) Representative: **Brown, Michael Stanley
Alpha and Omega,
Chine Croft,
East Hill
Ottery St. Mary,
Devon EX11 1PJ (GB)**

(56) References cited:
**WO-A-02/058557    US-A- 3 031 575
US-A- 3 226 545    US-B1- 6 621 086**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the Invention**

This invention relates to ionizing radiations.

Ionizing radiations are used, in particular, to cure cancer, i.e. by radiotherapy. The ionizing radiations have sufficient energy to break chemical bonds and separate electrons from their parent atoms and molecules in cancer cells, particularly in the nucleus containing DNA. The ultimate damage to the DNA leads to the death of the cancer cells.

However, gamma rays, alpha particles and beta particles used for medical purposes can be dangerous in that they can induce potential cancer when healthy cells around a cancer tumour are irradiated. In addition, X-rays which are used to medical imaging, for example, inspecting broken legs, can also induce potential cancer if a patient is over-exposed.

The absorbed dose of radiation is the amount of energy given to the medium, for example, a human exposed to radiation, per unit mass. It is normally measured in gray (Gy) defined as Joules/kg. Measurement of the absorbed dose due to radiation is the task of radiation dosimetry.

A number of different instruments have been used to measure the absorbed dose and are based on the detection of the physical or chemical changes caused by the radiation. For example, ionisation chambers measure the electrical charge produced by ionization of a gas inside a device called a detector. The measuring instruments typically provide an indirect determination of the dose.

Radiation dosimetry is needed in many areas, particularly in respect of cancer treatment using radiotherapy and in clinical diagnostic radiology.

As mentioned above, radiation therapy works by damaging the DNA of cancer cells causing them to die. Although normal cells are also sometimes damaged by the radiotherapy, healthy cells have the ability to repair themselves when subject to limited amounts of damage, while diseased cells are destroyed. External radiotherapy uses high energy rays (X-ray or gamma rays) or particle beams (protons or electrons) that are directed at the tumour to be treated, normally from several angles. Radiotherapy is normally given as a course of treatment spread over a number of days or weeks so as to allow the healthy cells around the tumour to recover from any damage that they may suffer.

It is, of course, important to ensure that the healthy cells are not subject to excessive damage and it is an object of the present invention to provide means for reducing the likelihood that such damage should occur.

In US Patent Specification No. 3,031,575 there is described an anthropometric phantom that comprises a simulation of at least a part of a human body and which includes cavities that contain a liquid which changes colour when exposed to radiation.

A specific liquid described in US Patent Specification No. 3,031,575 is a liquid gel mixture containing agar and potassium iodide.

A more specific object of the present invention is accordingly the provision of an improved anthropometric phantom.

**Summary of the Invention**

According to a first aspect of the present invention there is provided an anthropometric phantom that comprises a simulation of at least a part of a human body and which includes a number of cavities that contain a liquid that changes in colour when exposed to radiation, characterised in that the liquid in the cavities preferably comprises an aqueous solution of a chromate.

The aqueous chromate solution may be saturated with nitrous oxide. The aqueous solution may also contain a formate.

The main body of the phantom is preferably formed of polymethylmethacrylate, and may contain components formed of aluminium that simulate bones.

According to a second aspect of the present invention there is providing a method of assessing the doses of radiation resulting from the carrying out of a radiotherapy procedure that includes the use of an anthropometric phantom as defined above.

**Brief Description of the Drawings**

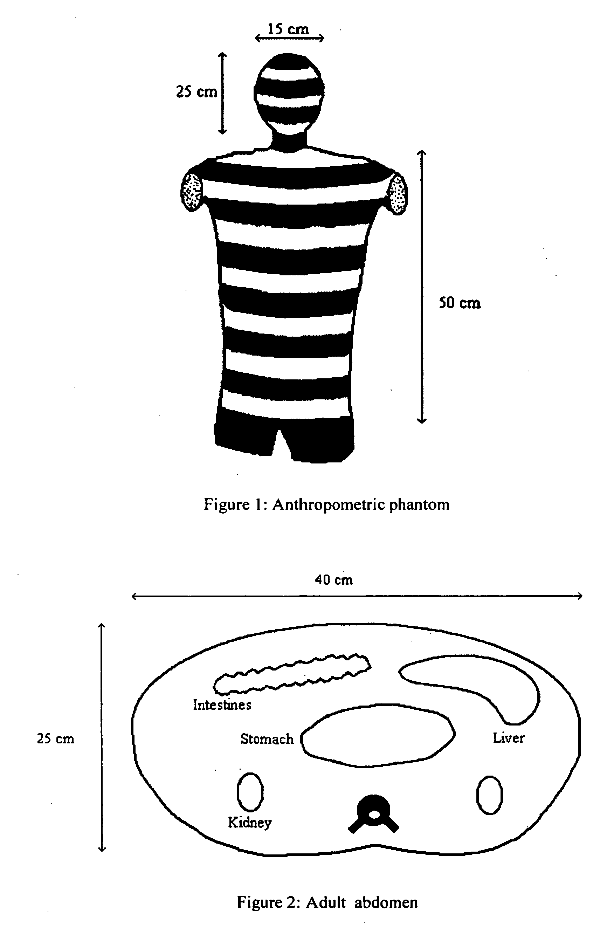

Figure 1 is a schematic view of an anthropometric phantom,

Figure 2 is a schematic horizontal sectional view of an adult abdomen,

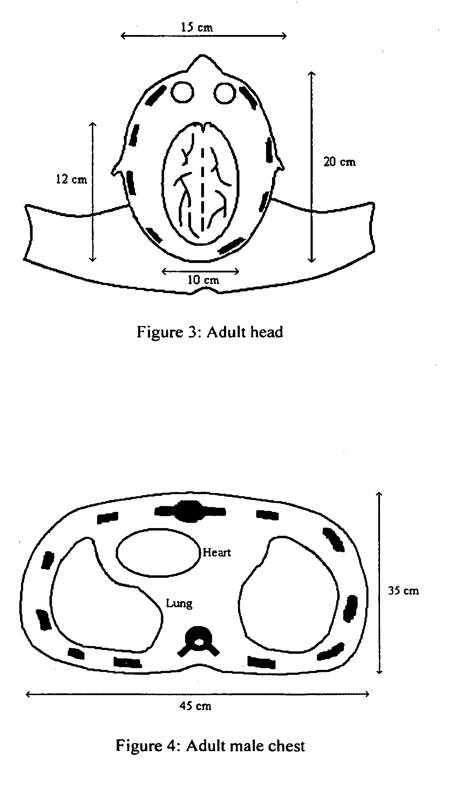

Figure 3 is a schematic horizontal sectional view of an adult head,

Figure 4 is a schematic horizontal sectional view of an adult male chest,

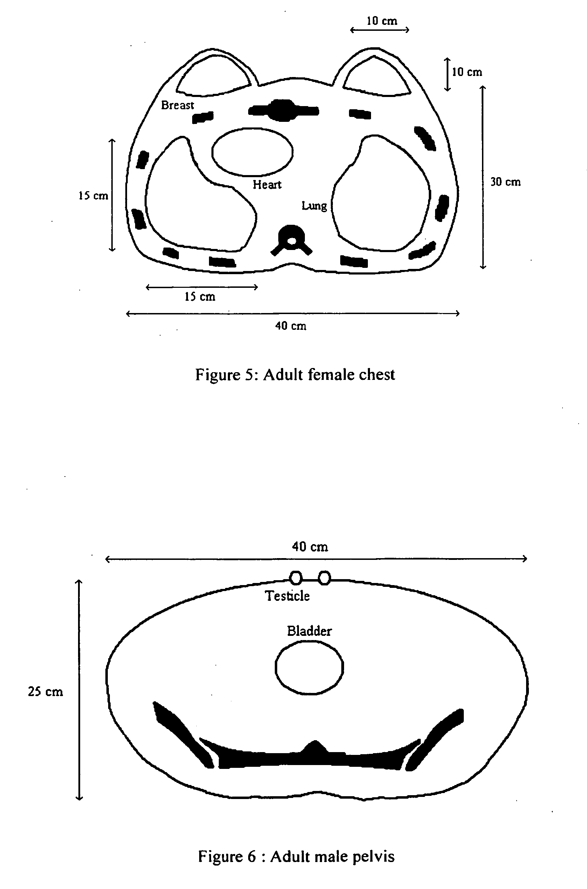

Figure 5 is a schematic horizontal sectional view of an adult female chest,

Figure 6 is a schematic horizontal sectional view of an adult male pelvis, and

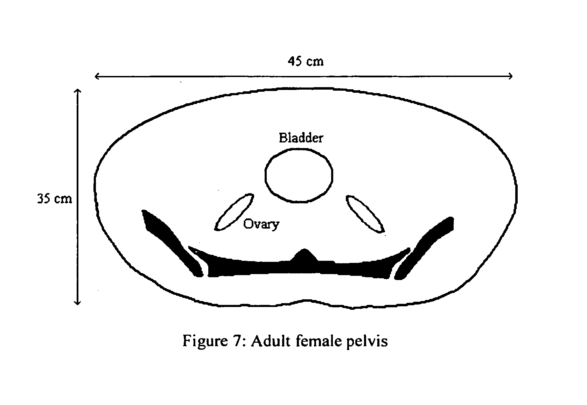

Figure 7 is a schematic horizontal sectional view of an adult female pelvis.

## Description of the Preferred Embodiment

The anthropometric phantom shown in the drawings is designed to provide a realistic representation of the human anatomical condition. It is produced from a material that has properties equivalent to those of human tissue, in terms of reaction to radiation. A suitable material is polymethylmethacrylate having a Specific Gravity of 1.19. As can be seen from the drawings, the phantom can simulate the head, neck, chest, abdomen and pelvic zone of a male or female. Components made of aluminium are contained within the structure made of polymethylmethacrylate and such components are designed to simulate different bones, for example, the cranium, spine and ribs.

Within the phantom there are cavities of different shapes and sizes designed to mimic selected body organs for in vivo dose measurements. For the particular phantoms shown in the drawings, the cavities that are provided represent the brain, eyes, thyroid gland, oesophagus, lungs, heart, stomach, liver, kidneys, rectum, bladder and gonads. If desired, the phantom model can be adjusted to match the sex and size of a particular patient. The cavities are filled with a chemical dosimeter solution and then, if desired, the chest, abdomen and pelvic zone of the phantom are covered with cloths to simulate clothing.

The completed anthropometric phantom is then placed on the treatment table of a radiotherapy machine and irradiated under the same protocol, i.e. the same dose for the same period of time, as a patient would be irradiated.

The chemical dosimeter solution with which the cavities are filled consists of a nitrous-oxide-saturated solution at pH 9.2 containing:-

a) $10^{-3}$ mol/dm of potassium chromate, and
b) $10^{-2}$ mol/dm of sodium formate.

When this solution absorbs radiation energy, for example, gamma or X-rays, or a beam of electrons or protons, the chromate solutions changes from a yellow colour to a greenish blue colour. The amount of chromate ion conversion is directly proportional to the amount of energy absorbed, i.e. to the total dose of radiation. The bleaching or disappearance of the chromate ions is determined spectrophotometrically, the maximum absorption wavelength of this ion being 370 nm. The degree of bleaching increases linearly with doses from 0.1 kGy to at least 10 kGy and is independent of dose rate up to 70 kGy/min.

Once the change in optical density (CIOD) has been determined by the measurements obtained using the spectrophotometer, the dose of radiation that has been received in a particular cavity can be calculated using the formula:-

$$\text{Dose} = 1.04 \times 10^3 \times (\text{CIOD}).$$

It is thus possible to assess whether, for a planned radiation treatment programme, the dose of radiation received in any of the cavities is above prescribed guidelines. The possibility of subjecting a patient to excessive doses of radiation can thus be reduced.

The present invention thus provides benchmarks for the quality assurance and safety control of current radiotherapy procedures. Dose mapping of the head, thorax and abdomen of the phantom is very useful for obtaining an optimum dose delivery for a real patient.

## Claims

1.   An anthropometric phantom that comprises a simulation of at least a part of a human body and which includes a number of cavities that contain a liquid that changes in colour when exposed to radiation, **characterised in that** the liquid in the cavities comprises an aqueous solution of a chromate.

2. An anthropometric phantom as claimed in Claim 1, in which the aqueous solution is saturated with nitrous oxide.

3. An anthropometric phantom as claimed in Claim 1 or Claim 2, in which the aqueous solution also contains a formate.

4. An anthropometric phantom as claimed in any one of the preceding claims, in which the main body of the phantom is formed of polymethylmethacrylate.

5. An anthropometric phantom as claimed in Claim 4, which contains components formed of aluminium that simulate bones.

6. A method of assessing the doses of radiation resulting from the carrying out of a radiotherapy procedure that includes the use of an anthropometric phantom as claimed in any one of the preceding claims.


**Patentansprüche**

1. Anthropometrisches Phantom, das eine Simulation zumindest eines Teils eines menschlichen Körpers umfasst und eine Anzahl von Hohlräumen aufweist, die eine Flüssigkeit enthalten, die bei Strahlenexposition ihre Farbe ändert, **dadurch gekennzeichnet, dass** die Flüssigkeit in den Hohlräumen eine wässrige Chromatlösung umfasst.

2. Anthropometrisches Phantom nach Anspruch 1, bei dem die wässrige Lösung mit Stickoxid gesättigt ist.

3. Anthropometrisches Phantom nach Anspruch 1 oder Anspruch 2, bei dem die wässrige Lösung auch ein Formiat enthält.

4. Anthropometrisches Phantom nach einem der vorhergehenden Ansprüche, bei dem der Hauptkörper des Phantoms aus Polymethylmethacrylat geformt ist.

5. Anthropometrisches Phantom nach Anspruch 4, das Aluminiumkomponenten enthält, die Knochen simulieren.

6. Verfahren zur Beurteilung der Strahlendosen, die sich aus der Durchführung eines Strahlentherapieverfahrens ergeben, bei dem ein anthropometrisches Phantom nach einem der vorhergehenden Ansprüche verwendet wird.


**Revendications**

1. Fantôme anthropométrique qui comprend une simulation d'au moins une partie d'un corps humain et qui inclut un certain nombre de cavités qui contiennent un liquide qui change de couleur lorsqu'il est exposé à des radiations, **caractérisé en ce que** le liquide dans les cavités comprend une solution aqueuse d'un chromate.

2. Fantôme anthropométrique selon la revendication 1, dans lequel la solution aqueuse est saturée d'oxyde nitreux.

3. Fantôme anthropométrique selon la revendication 1 ou la revendication 2, dans lequel la solution aqueuse contient également un formiate.

4. Fantôme anthropométrique selon l'une quelconque des revendications précédentes, dans lequel le corps principal du fantôme est formé de polyméthacrylate de méthyle.

5. Fantôme anthropométrique selon la revendication 4, qui contient des composants formés d'aluminium qui simulent les os.

6. Méthode d'évaluation des doses de radiation qui résultent de la mise en oeuvre d'un procédé de radiothérapie qui inclut l'utilisation d'un fantôme anthropométrique selon l'une quelconque des revendications précédentes.

15 cm

25 cm

50 cm

Figure 1: Anthropometric phantom

40 cm

25 cm

Intestines

Stomach

Liver

Kidney

Figure 2: Adult abdomen

5

Figure 3: Adult head

Figure 4: Adult male chest

Figure 5: Adult female chest

Figure 6 : Adult male pelvis

Figure 7: Adult female pelvis

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 3031575 A